# EUROPÄISCHE PATENTANMELDUNG

(11) **EP 1 803 397 A2**
(43) Veröffentlichungstag der Anmeldung: **04.07.2007**
(21) Anmeldenummer: 06018142.7
(22) Anmeldetag: 30.08.2006
(51) Int. Cl.: A61B 5/22, A61B 5/11

(54) **Monitoring System**

(30) Priorität: 13.09.2005 DE 102005043647
(71) Anmelder: Actimon GmbH & Co. KG, 69190 Walldorf (DE)
(72) Erfinder: Wegertseder, Dominik, 85540 Haar (DE); Schweizer, Thomas, 85560 Ebersberg (DE)
(74) Vertreter: Müller-Boré & Partner Patentanwälte

(57) **Zusammenfassung**

Monitoring-System, insbesondere Adipositas-Monitoring-System, zur Überwachung der Aktivität eines Lebewesens, umfassend zumindest einen Bewegungssensor, der am Lebewesen anordenbar und ausgelegt ist, alltägliche Bewegungsmuster zu erfassen, zumindest eine Feedback-Einrichtung, welche am Lebewesen oder diesem benachbart angeordnet ist, und zumindest eine Datenverarbeitungseinrichtung, welche mobil ausgebildet ist und mit dem Bewegungssensor der Feedback-Einrichtung in Signalverbindung steht, wobei die Verarbeitungseinrichtung eine Datenbank, welche Daten in Form von Bewegungsmustern umfaßt, einen Komparator, der zum Vergleich der vom Bewegungssensor ermittelten Bewegungsmuster mit den vorgegebenen Bewegungsmustern der Datenbank ausgelegt ist, und eine Prozessoreinheit aufweist, welche mit dem Komparator in Signalverbindung steht und ausgelegt ist, eine Steuereinheit der Datenverarbeitungseinrichtung in Abhängigkeit des Vergleichsergebnisses zu steuern bzw. zu regeln, wobei die Steuereinheit ausgelegt ist, innerhalb einer vorbestimmbaren Zeitdauer in Abhängigkeit der Steuerung bzw. Regelung durch die Prozessoreinheit eine für das Lebewesen wahrnehmbare Reaktion über die Feedback-Einrichtung auszuüben.

## Beschreibung

Die vorliegende Erfindung bezieht sich auf ein Monitoring-System, insbesondere Adipositas-Monitoring-System, sowie auf ein Monitoring-Verfahren, insbesondere Adipositas-Monitoringverfahren, welches zur Überwachung der Aktivität eines Lebewesens ausgelegt ist.

Adipositas oder Fettsucht ist ein sozioökonomisch ernstzunehmendes Problem in den westlichen Industrienationen. Übergewicht aufgrund von Adipositas entsteht insbesondere aufgrund falscher Ernährung und wird zudem durch Bewegungsarmut verstärkt, wobei zunehmender Bewegungsmangel insbesondere bei Kindern und Jugendlichen verstärkt zu beobachten ist, verursacht durch steigenden TV-Konsum und Computerspiele. Bei dieser Gruppe sind hohe Folgekosten in der Gesundheitsversorgung in den nächsten Jahren zu erwarten. Da jedoch bei Jugendlichen die Möglichkeit besteht, den Lebensstil langfristig umzustellen, wurde in der DE 196 03 237 A1 ein Trainingsgerät vorgeschlagen, welches ein Körperübungsgerät, wie z.B. einen Ergometer oder ein Heimfahrrad, zur Messung der körperlich geleisteten Arbeit und Wägezellen zur Gewichtsmessung einsetzt, um die durch die Wägezellen ermittelten Daten am Trainingsgerät auszuwerten. Problematisch bei derartigen Vorrichtungen ist jedoch, daß diese aufwendig zu bedienen sind und aufgrund ihrer stationären (d.h. ortsfesten) Anordnung nur singuläre (Einzel-)Messungen ermöglichen.

Es ist somit Aufgabe der vorliegenden Erfindung, ein Monitoring-System sowie ein Monitoring-Verfahren zur Überwachung der Aktivität eines Lebewesens, insbesondere als Adipositas-Monitoring-System und Adipositas-Monitoring-Verfahren, vorzusehen, welche alltäglich einsetzbar sind, einfach zu bedienen sind und im täglichen Ablauf intgrierbar sind.

Diese Aufgabe wird durch ein Monitoring-System, insbesondere Adipositas-Monitoring-System, zur Überwachung der Aktivität eines Lebewesens mit den Merkmalen des Anspruchs 1 sowie durch ein Monitoring-Verfahren, insbesondere Adipositas-Monitoring-Verfahren, zur Überwachung der Aktivität eines Lebewesens mit den Merkmalen des Anspruchs 15, gelöst. Bevorzugte Ausführungsformen sind Gegenstand der abhängigen Unteransprüche.

Erfindungsgemäß ist ein Monitoring-System, insbesondere Adipositas-Monitoring-System, zur Überwachung der Aktivität eines Lebewesens, vorgesehen, umfassend zumindest einen Bewegungssensor, der am Lebewesen anordenbar und ausgelegt ist, alltägliche Bewegungsmuster zu erfassen, zumindest eine Feedback-Einrichtung, welche am Lebewesen oder diesem benachbart angeordnet ist, und zumindest eine Datenverarbeitungseinrichtung, welche mobil ausgebildet ist und mit dem Bewegungssensor der Feedback-Einrichtung in Signalverbindung steht, wobei die Verarbeitungseinrichtung eine Datenbank, welche Daten in Form von Bewegungsmustern umfaßt, einen Komparator, der zum Vergleich der vom Bewegungssensor ermittelten Bewegungsmuster mit den vorgegebenen Bewegungsmustern der Datenbank ausgelegt ist, und eine Prozessoreinheit aufweist, welche mit dem Komparator in Signalverbindung steht und ausgelegt ist, eine Steuereinheit der Datenverarbeitungseinrichtung in Abhängigkeit des Vergleichsergebnisses zu steuern bzw. zu regeln, wobei die Steuereinheit ausgelegt ist, innerhalb einer vorbestimmbaren Zeitdauer in Abhängigkeit der Steuerung bzw. Regelung durch die Prozessoreinheit eine für das Lebewesen wahrnehmbare Reaktion über die Feedback-Einrichtung auszuüben.

Der zumindest eine Bewegungssensor ist vorteilhafterweise in einer kompakten Bauart ausgeführt und eigenständig am Objekt angeordnet, d.h. unabhängig von der Position der weiteren Elemente des Monitoring-Systems positionierbar. Infolgedessen ist es möglich, den Bewegungssensor derart zu positionieren, daß die Bewegungsabläufe des Lebewesens nicht negativ beeinflußt werden. Der Bewegungssensor ist im wesentlichen mobil, d.h. tragbar ausgebildet, d.h. er weist vorzugsweise ein Gewicht kleiner als 1 kg, besonders bevorzugterweise ein Gewicht kleiner 200 g auf. Der Bewegungssensor ist derart ausgelegt, daß er fähig ist, alltägliche Bewegungsmuster zu erfassen. Zu alltäglichen Bewegungsmustern zählen insbesondere Bewegungen bzw. Positionen wie Stehen, Sitzen, Gehen und Laufen. Insbesondere ist der Bewegungssensor derart ausgebildet, daß die von ihm erfaßten Daten im wesentlichen mit dem Energieverbrauch des Lebewesens in Zusammenhang stehen, da die Bewegung eines Lebewesens einen Energieverbrauch mit sich bringt. Die Feedback-Einrichtung kann einerseits am Lebewesen selbst oder diesem benachbart angeordnet sein. In anderen Worten ist es möglich, die Feedback-Einrichtung an einem dem Lebewesen asoziierten zweiten Gegenstand bzw. an einer mit dem Lebewesen in Verbindung stehenden Person anzuordnen, welche Empfänger des Feedbacks sein soll. Selbstverständlich kann die Feedback-Einrichtung ebenfalls am Lebewesen selbst angeordnet sein, beispielsweise in Form einer Armbanduhr, eines Handys, eines PDA's oder eines Laptops. Durch eine Signalverbindung bzw. einen Signalkontakt der Datenverarbeitungseinrichtung mit dem Bewegungssensor einerseits und der Feedback-Einrichtung andererseits ist es möglich, eine Vielzahl von verschiedenen Daten zwischen diesen Elementen zu übertragen. Zu diesen Daten zählen insbesondere Daten, welche mit dem durch den Bewegungssensor erfaßten Bewegungsmuster in Verbindung stehen (sogenannte "erfaßte Bewegungsmusterdaten"). Die Bewegungsmusterdaten können dabei sowohl auf Einzelmessungen beruhen als auch dynamischen Sequenzen entsprechen. In anderen Worten können diese Daten einerseits Einzel-Daten sein oder ganze Datensätze. In diesem Zusammenhang bedeuten Einzel-Daten diejenigen Daten, mittels derer sich beispielsweise die Bewegung bzw. Position eines Objekts zu einem bestimmten Zeitpunkt feststellen läßt. Mittels Datensätzen ist es darüber hinaus möglich, einen spezifischen Bewegungsablauf quantitativ und qualitativ zu erfassen, d.h. eine über einen vorbestimmten Zeitraum definierte Aneinanderreihung von einzelnen Positionen. Infolgedessen ist es vorteilhafterweise möglich, Zeiten bzw. Zeitabläufe zu messen und in dem Monitoring-System zu integrieren. In der Datenverarbeitungseinrichtung ist eine Datenbank vorgesehen, welche Daten in Form von vorgegebenen Bewegungsmustern umfaßt. Die Daten in Form von vorgegebenen Bewegungsmustern können einerseits in Form von repräsentativen ldealwerten (d.h. idealen Bewegungsabläufen) und andererseits als qualitative Vorgabe von einer generellen Bewegung oder spezifizierten Bewegungsmustern in einem vorbestimmten Zeitraum vorgesehen sein. Durch den Komparator werden vorteilhafterweise die durch den Bewegungssensor erfaßten Bewegunsgmsuterdaten mit den entsprechenden Referenzdaten, welche in der Datenbank der Datenverarbeitungseinrichtung abgelegt sind, verglichen und, ausgehend von diesem Vergleich, über geeignete Rechenoperationen entsprechende Ergebnisse ausgegeben. Der Komparator ist also so ausgebildet, daß er vorteilhafterweise die Qualität und/oder die Quantität der Bewegungsmuster vergleicht, so daß sich anhand dieses Vergleichsergebnisses eine Aussage darüber treffen läßt, inwieweit das Bewegungsmuster zu einem Energieverbrauch des Lebewesens geführt hat. Abhängig von den Vergleichsergebnissen des Komparators ist die Prozessoreinheit der Datenverarbeitungseinrichtung vorteilhafterweise ausgebildet, eine Steuereinheit zu steuern bzw. zu regeln, welche in Signalverbindung mit der Feedback-Einrichtung steht. Über diese Feedback-Einrichtung wird eine für das Lebewesen wahrnehmbare Reaktion ausgeführt. Die Reaktion kann derart gestaltet werden, daß sie vom Lebewesen anhand von Bildern, Text und/oder akustisch negativ ausgelegt wird, wenn die in der Datenbank abgelegten Referenzdaten nicht erreicht bzw. nicht eingehalten werden, d.h. ein nicht ausreichender Energieverbrauch stattgefunden hat. Auf der anderen Seite ist die Feedback-Einrichtung ausgebildet, ein positives Feedback in Form von Belobigungen oder Belohnungen auszugeben, wenn die Bewegungsmusterdaten quantitativ und/oder qualitativ den in der Datenbank abgelegten Bewegungsmusterdaten entsprechen oder diese übertreffen. Vorteilhafterweise ist die Prozessoreinheit bzw. Steuereinheit derart ausgelegt, daß die Reaktion über die Feedback-Einrichtung innerhalb einer vorbestimmten Zeitdauer ausgeführt wird. Infolgedessen kann die Reaktion zeitnah bzw. zeitlich unmittelbar erfolgen, insbesondere innerhalb weniger Sekunden, vorzugsweise weniger als 10 Sekunden, besonders bevorzugterweisen weniger als 1 Sekunde. Es ist ebenfalls möglich, die Reaktion turnusmäßig zu einem bestimmten Tageszeitpunkt auszuüben, beispielsweise jeden Abend.

Vorzugsweise weist der Bewegungssensor Mittel zur Erfassung einer Beschleunigung auf, um über eine zeitliche Integration ein Bewegungsmuster zu erfassen. Der Bewegungssensor kann somit als Beschleunigungs- und/oder Drehratensensor ausgebildet sein, der vorzugsweise jeweils in drei Achsen bzw. Achsrichtungen Drehraten bzw. Beschleunigungen messen kann. Die drei Achsrichtungen sind vorteilhafterweise so ausgebildet, daß diese den dreidimensionalen Raum aufspannen, um eine Bewegung in jegliche Raumrichtung zu erfassen. Vorzugsweise stehen die Achsrichtungen senkrecht zueinander. Zusätzlich oder alternativ weist der. Bewegungssensor Mittel zur Erfassung einer Geschwindigkeit auf, um ein Bewegungsmuster zu erfassen. Über die Geschwindigkeit kann somit in zeitlicher Abhängigkeit vorteilhafterweise die während der Bewegung zurückgelegte Wegstrecke ermittelt werden, so daß der Energieverbrauch ermittelt werden kann. Darüber hinaus kann der Bewegungssensor zusätzlich oder alternativ auch Mittel zur Erfassung der Position aufweisen, über welche über eine zeitliche Integration bei Positionsänderung ein Bewegungsmuster erfaßt wird.

In einer weiteren bevorzugten Ausführungsform weisen die Datenverarbeitungseinrichtung und/oder der Bewegungssensor eine Zeiterfassungseinrichtung auf, um die Bewegungsmuster über einen vorbestimmten oder vorbestimmbaren Zeitraum zu erfassen. Die Zeiterfassungseinrichtung ist in dem erfindungsgemäßen Monitoring-System vorteilhafterweise einerseits als "Stoppuhr" zu verstehen und andererseits ebenfalls als Signalgeber, der Zeitsignale an die entsprechenden Einrichtungen abgibt. Mittels der Zeiterfassungseinrichtung kann somit die Bewegung des Lebewesens über einen vorbestimmten Zeitraum erfaßt werden, beispielsweise über Stunden, Tage oder Monate, um somit den Energieverbrauch des Lebewesens beziffern zu können. Andererseits kann die Zeiterfassungseinrichtung als Signalgeber agieren, so daß, in Abhängigkeit von den Zeitsignalen und einer vom Bewegungssensor gemessenen Beschleunigung, die Wegstrecke, Geschwindigkeit sowie der Geschwindigkeitsverlauf (d.h. die Beschleunigung) errechnet werden kann.

Zweckmäßigerweise weist das Monitoring-System eine Eingabeeinrichtung zur Eingabe von mit Bewegungsmustern in Zusammenhang stehenden Daten auf. Derartige Daten können einerseits Zeitspannen, innerhalb welcher gewisse Bewegungen ausgeführt wurden, sein, andererseits die Art und Weise der Bewegung. Darüber hinaus können über die Eingabe vorteilhafterweise ebenfalls Parameter bezüglich der Auswahl und Zusammenstellung der Nahrungszufuhr, d.h. das Ernährungsverhalten, eingegeben werden. Ferner ist die Eingabeeinrichtung vorteilhafterweise ausgebildet, weitere individuelle Parameter bezüglich des Lebewesens über die Eingabe aufzunehmen, beispielsweise die Größe, das Gewicht, das Alter oder den Gesundheitszustand. Ebenso können über die Eingabeeinrichtung Schwierigkeitsstufen zu den Bewegungsabläufen bzw. -mustern eingegeben werden, beispielsweise in standardisierter Form oder über Normwerte, welche in einer entsprechenden Datenbank abgelegt sind.

Vorteilhafterweise weist das Monitoring-System eine Speichereinrichtung auf, die vorzugsweise in der Datenverarbeitungseinrichtung, dem Bewegungssensor und/oder der Feedback-Einrichtung vorgesehen ist. Die Speichereinrichtung kann zweckmäßigerweise die vom Bewegungssensor eingelesenen bzw. erfaßten Daten, die in der Datenbank abgelegten Referenzwerte und die entsprechenden Verarbeitungsdaten bzw. -programme der Prozessoreinheit speichern. Als Speichermedium können hier insbesondere sämtliche flüchtigen und nicht-flüchtigen elektronischen Halbleiterspeicher, insbesondere DRAM's und Flash-Speicher, verwendet werden.

Bevorzugt umfaßt die Feedback-Einrichtung optische, akustische und/oder kinästhetische Ausgabemittel, um eine Reaktion auszuüben. Derartige optischen und/oder akustischen Ausgabemittel können beispielsweise ein Lautsprecher, Kopfhörer, Display oder Leuchtdioden sein. Kinästhetische Ausgabemittel sind vorteilhafterweise ausgebildet, eine Bewegung oder einen Druck bzw. eine sonstige Kraft auf einen Körper, vorzugsweise das Lebewesen, auszuüben.

Weiterhin bevorzugt sind zumindest die Feedback-Einrichtung und die Datenverarbeitungseinrichtung integral ausgebildet. In anderen Worten sind zumindest die Feedback-Einrichtung und die Datenverarbeitungseinrichtung in einem gemeinsamen Gehäuse angeordnet, beispielsweise einem Handy oder PDA. Es ist jedoch ebenfalls möglich, sämtliche Elemente des Monitoring-Systems integral auszubilden, so daß die Handhabbarkeit des Monitoring-Systems vorteilhafterweise erleichtert wird.

In einer bevorzugten Ausführungsform weist das Monitoring-System weiterhin eine Auswerteeinrichtung auf, welche mit der Datenverarbeitungseinrichtung in Signalverbindung steht oder bringbar ist, um die erfaßten Bewegungsmuster und/oder die Vergleichsergebnisse weiter zu verarbeiten. Die Auswerteeinrichtung ist vorteilhafterweise ortsfern zu dem Lebewesen bzw. zu der Datenverarbeitungseinrichtung angeordnet. In anderen Worten ist die Auswerteeinrichtung nicht in mittelbarem oder unmittelbarem körperlichen Kontakt zu dem Lebewesen vorgesehen sondern so angeordnet, daß die Bewegung bzw. die Position des Lebewesens von der Auswerteeinrichtung unabhängig möglich ist. Die Signalverbindung kann vorteilhafterweise über Funkübertragungsmittel erfolgen, welche vorzugsweise auf einer gesicherten Signalverbindung basieren. Somit könnte die Auswerteeinrichtung mittels einer Funkverbindung an einem zentralen Ort, beabstandet von dem Lebewesen, angeordnet sein, vorzugsweise im Bereich von kleiner einem Meter bis mehreren Kilometer. Eine Übertragung der erfaßten Bewegungsmuster und/oder der Vergleichsergebnisse an die Auswerteeinrichtung kann ebenfalls über das Internet erfolgen. Die Signalverbindung zwischen Auswerteeinrichtung und Datenverarbeitungseinrichtung kann kontinuierlich erfolgen oder auch zeitweise, beispielsweise turnusmäßig zu einem bestimmten Tageszeitpunkt.

Zweckmäßigerweise sind die Datenverarbeitungseinrichtung und/oder die Feedback-Einrichtung ausgelegt, von der Auswerteeinrichtung Daten zu empfangen. In anderen Worten ist die Signalverbindung zwischen Auswerteeinrichtung und Datenverarbeitungseinrichtung bzw. Feedback-Einrichtung bidirektional ausgebildet, d.h. ein Datenfluß kann in beide Richtungen erfolgen. Somit ist es über das erfindungsgemäße Monitoring-System möglich, die Datenverarbeitungseinrichtung von einer - vorzugsweise ortsfernen - Auswerteeinrichtung zu steuern bzw. zu regeln oder der Datenverarbeitungseinrichtung zusätzliche Daten zu übermitteln. Dies könnten beispielsweise Daten sein, welche mit der Belohnung des Lebewesens bei einem positiven Feedback in Zusammenhang stehen. Konsequenterweise kann die Datenverarbeitungseinrichtung entsprechend auch gesteuert werden, falls ein negatives Feedback ausgegeben werden soll.

Bevorzugterweise weist die Datenverarbeitungseinrichtung eine weitere Datenbank in Form einer Gesundheitsdatenbank auf, welche ausgelegt ist, die durch den Bewegungssensor erfaßten Bewegungsmuster medizinisch zu klassifizieren und/oder zu speichern. Die weitere Datenbank ist hierbei vorteilhafterweise ausgelegt, die klassifizierten Daten an die Auswerteeinrichtung zu übermitteln. Vorteilhafterweise ist die Gesundheitsdatenbank unabhängig von der Funktionsweise der Feedback-Einrichtung ausgebildet und läuft für das Lebewesen "unsichtbar" im Hintergrund mit, um Daten entsprechend aufzuzeichnen und zu klassifizieren. Über die Auswerteeinrichtung kann es beispielsweise möglich sein, daß Dritte (z.B. ein Arzt) auf die Gesundheitsdatenbank zugreifen können, um entsprechend die Vergleichsergebnisse und/oder die erfaßten Bewegungsmuster bzw. entsprechend klassifizierten Daten zu erfassen.

Zweckmäßigerweise weisen die Datenverarbeitungseinrichtung, der Bewegungssensor, die Feedback-Einrichtung und/oder die Auswerteeinrichtung Funkübertragungsmittel auf. Es ist somit möglich, die Signalübertragung zwischen den einzelnen Elementen drahtlos zu gestalten, wobei beispielsweise die drahtlose Übertragung mittels "blue tooth" oder anderen standardisierten oder proprietären Verfahren erfolgen kann.

In einer vorteilhaften Ausführungsform des erfindungsgemäßen Monitoring-Systems kann die Bewegung von Lebewesen mittels Bewegungssensoren überwacht werden, wobei der gemessene Wert als Input für ein Spiel dient. Das Spiel kann derart gestaltet werden, daß verschiedene bzw. höhere Level des Spiels bzw. die Erzielung von Gewinnen nur durch eine kontinuierliche bzw. in Qualität und Quantität vorbestimmbare Bewegung erreicht werden. Somit wird vorteilhafterweise erreicht, daß Bewegungen mit Spielfreude und dem Anreiz weiterer Level gefördert wird, und Bewegung spielerisch in dem täglichen Lebensrhythmus integriert wird. Infolgedessen wird auf der Feedback-Einrichtung ein Spiel oder sonstiger Gewinn bzw. Belohnung für das Lebewesen dargestellt. Gewinne könnten beispielsweise erfolgen, indem auf der Feedback-Einrichtung Internet-Freischaltcodes vorgesehen werden, mittels welchen für einen bestimmten Zeitraum im Internet gesurft werden könnte. Entsprechendes gilt für Fernsehen, Computerspiele, etc. Selbstverständlich kann das Spiel derart implementiert werden, daß bei negativen Vergleichsergebnissen bzw. den erfaßten Bewegungsmustern, die unter dem vorgegebenen Wert liegen, ein Rückfall in ein niedrigeres Spiellevel forciert wird, bzw. Fernseh-, Spiel- oder Internetzeiten verringert werden. Ebenfalls denkbar ist die Kreation eines Tamagotschi-ähnlichen Lebewesens, so daß eine Interaktion zwischen Lebewesen und dem erfindungsgemäßen Monitoring-System auf spielerische Art und Weise eine hohe Qualität und Quantität an Bewegungen bewirkt.

Weiterhin erfindungsgemäß ist ein Monitoringverfahren, insbesondere Adipositas-Monitoringverfahren, zur Überwachung der Aktivität eines Lebewesens vorgesehen, umfassend die Schritte: Erfassen eines alltäglichen Bewegungsmusters des Lebewesens mittels eines am Lebewesen anordenbaren Bewegungssensors; Übertragen von dem erfaßten Bewegungsmuster entsprechenden Daten an eine mobile Datenverarbeitungseinrichtung; Vergleichen der erfaßten Bewegungsmusterdaten mit in einer Datenbank der Datenverarbeitungseinrichtung abgelegten vorgegebenen Bewegungsmusterdaten durch einen Komparator; Steuern bzw. Regeln einer Steuereinheit der Datenverarbeitungseinrichtung in Abhängigkeit des Vergleichsergebnisses; Ausüben einer für das Lebewesen wahrnehmbaren Reaktion durch die Feedback-Einrichtung innerhalb einer vorbestimmbaren Zeitdauer über eine Steuereinheit in Abhängigkeit der Steuerung bzw. Regelung durch die Prozessoreinheit.

Bevorzugterweise weist das Monitoringverfahren den Schritt auf: Übertragen der erfaßten Bewegungsmusterdaten und/oder des Vergleichsergebnisses an eine mit der Datenverarbeitungseinrichtung in Signalverbindung stehende Auswerteeinrichtung. Besonders bevorzugterweise weist das Monitoringverfahren den Schritt auf: Übertragen von Daten von der Auswerteeinrichtung an die Datenverarbeitungseinrichtung und/oder Feedback-Einrichtung. Infolgedessen wird zwischen der Auswerteeinrichtung und der Datenverarbeitungseinrichtung bzw. Feedback-Einrichtung eine bidirektionale Datenübertragung geschaffen.

Vorteilhafterweise weist das Monitoringverfahren weiterhin den Schritt auf: Medizinisch Klassifizieren und/oder Speichern der erfaßten Bewegungsmusterdaten in einer Gesundheitsdatenbank der Datenverarbeitungseinrichtung.

Die mit Bezug auf das erfindungsgemäße Monitoring-System beschriebenen Merkmale und Vorteile sind entsprechend auch für das erfindungsgemäße Monitoring-Verfahren zutreffend.

Die Erfindung wird nachfolgend anhand begleitender Zeichnungen bevorzugter Ausführungsformen beispielhaft beschrieben. Es zeigt:
Fig. 1 ein Diagramm einer bevorzugten Ausführungsform des erfindungsgemäßen Monitoring-Systems;
Fig. 2 ein Diagramm einer zweiten bevorzugten Ausführungsform des erfindungsgemäßen Monitoring-Systems; und
Fig. 3 ein Diagramm einer dritten bevorzugten Ausführungsform des erfindungsgemäßen Monitoring-Systems.

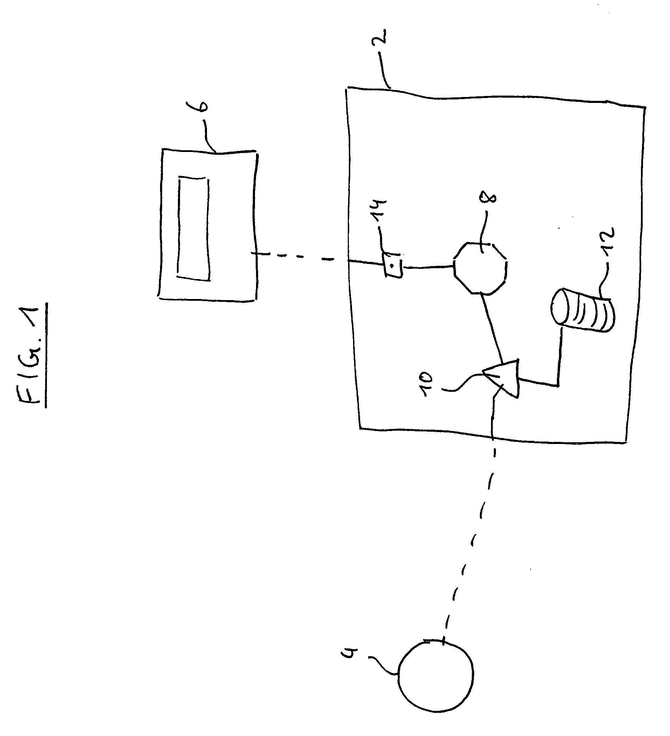

In Fig. 1 ist beispielhaft ein Diagramm einer ersten Ausführungsform des erfindungsgemäßen Monitoring-Systems dargestellt. Das Monitoring-System umfaßt eine Datenverarbeitungseinrichtung 2, welche mit zumindest einem Bewegungssensor 4 in Signalverbindung steht. Ferner steht die Datenverarbeitungseinrichtung 2 ebenfalls mit einer Feedback-Einrichtung 6 in Signalverbindung. Die Datenverarbeitungseinrichtung 2, Bewegungssensor 4 und Feedback-Einrichtung 6 können als getrennte Elemente ausgebildet sein, jedoch ebenfalls integral in einer Einheit angeordnet sein. Beispielsweise könnte die Datenverarbeitungseinrichtung 2 und die Feedback-Einrichtung 6 in einem Handy, PDA oder Laptop vorgesehen sein, welche mit wenigstens einem Bewegungssensor 4 in Signalverbindung stehen. Es versteht sich, daß ebenfalls der Bewegungssensor 4 Teil der integralen Einheit von Datenverarbeitungseinrichtung 2 und Feedback-Einrichtung 6 sein kann.

Die Datenverarbeitungseinrichtung 2 weist eine Prozessoreinheit 8, einen Komparator 10, eine Datenbank 12 sowie eine Steuereinheit 14 auf. Die Datenverarbeitungseinrichtung 2 ist vorteilhafterweise mobil ausgebildet, d.h. von einem Lebewesen tragbar. In anderen Worten weist die Datenverarbeitungseinrichtung 2 ein Gewicht kleiner als 10 kg, vorzugsweise kleiner als 1 kg, besonders bevorzugterweise ein Gewicht kleiner als 200 g, auf. In ihren geometrischen Abmessungen ist die Datenverarbeitungseinrichtung 2 kleiner als 0,5 m³, vorzugsweise kleiner als 0,1 m³ und ganz besonders bevorzugterweise kleiner als 0,05 m³. Der Komparator 10 ist ausgelegt, die von dem zumindest einen Bewegungssensor 4 erfaßten Daten aufzunehmen. Diese erfaßten Daten werden mittels des Komparators 10 mit in der Datenbank 12 abgelegten Daten verglichen. In der Datenbank 12 sind Daten in Form von vorgegebenen Bewegungsmustern, bzw. idealen Bewegungsmustern vorgegeben. Die Daten können also als Bewegungsmuster hinsichtlich ihrer Qualität als auch ihrer Quantität über einen vorbestimmten Zeitraum vorgesehen sein. Infolgedessen ist der Komparator 10 vorteilhafterweise ausgelegt, die vom Bewegungssensor 4 erfaßten Daten mit den in der Datenbank 12 abgelegten Daten hinsichtlich ihrer Qualität sowie Quantität zu vergleichen. Die Datenbank 12 umfaßt somit insbesondere Daten in Form von SollWerten. Das vom Komparator 10 ermittelte Vergleichsergebnis wird der Prozessoreinheit 8 übermittelt, in welcher zumindest das Vergleichsergebnis weiterverarbeitet wird. In Abhängigkeit vom Wert des Vergleichsergebnisses ist die Prozessoreinheit 8 ausgelegt, die Steuereinheit 14 zu steuern bzw. zu regeln, welche ihrerseits wiederum die Feedback-Einrichtung 6 steuert bzw. regelt. Die Prozessoreinheit 8 ist somit ausgelegt, in Abhängigkeit vom Vergleichsergebnis des Komparators 10 der Steuereinheit 14 ein Signal zuzuführen, in dessen Abhängigkeit die Steuereinheit 14 die Feedback-Einrichtung 6 steuert bzw. regelt, d.h. entweder ein positives oder negatives Feedback dem Lebewesen vermittelt. Vorteilhafterweise können Prozessoreinheit 8 und/oder Steuereinheit 14 nicht nur lediglich ein positives bzw. negatives Feedback bewirken, sondern ebenfalls Zwischenstufen davon.

Die Feedback-Einrichtung 6 ist vorteilhafterweise ausgelegt, ein Feedback akustisch, optisch, kinästhetisch oder in sonstiger Art und Weise zu vermitteln. Hierzu kann beispielsweise in der Feedback-Einrichtung 6 ein Display vorgesehen sein, beispielsweise in Form einer Uhr. Die Feedback-Einrichtung 6 kann somit als Teil einer Armbanduhr ausgebildet sein. Selbstverständlich kann die Feedback-Einrichtung 6 ebenfalls als PC, Playstation, PDA, Handy oder als sonstige mit einem Display oder einer Anzeigeeinrichtung versehene Einrichtung ausgebildet sein.

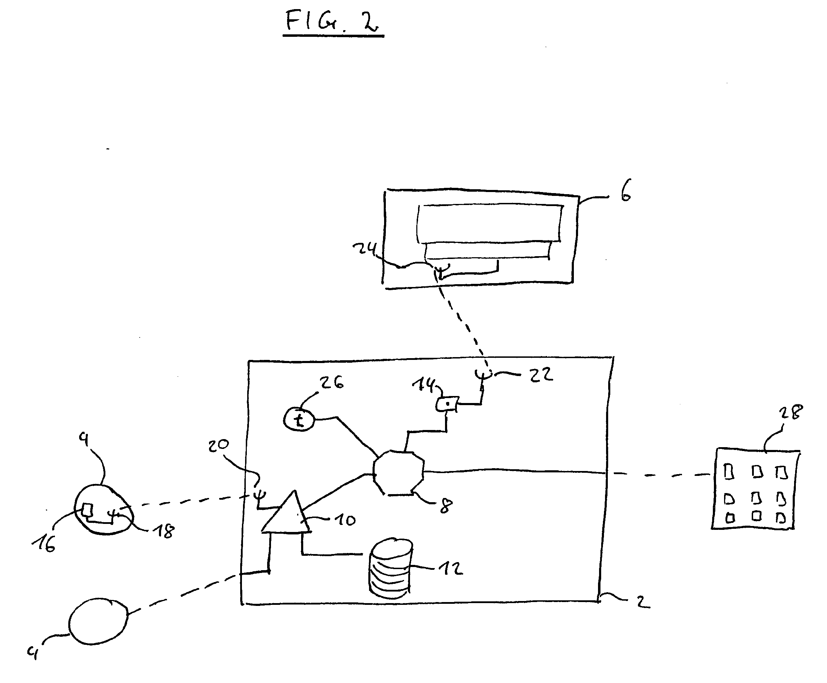

Der Bewegungssensor 4 ist ausgebildet, alltägliche Bewegungsmuster zu erfassen. Zu den alltäglichen Bewegungsmustern zählen insbesondere Bewegungen des Lebewesens wie Stehen, Sitzen, Gehen, Laufen oder Liegen. Insbesondere sollen durch den Bewegungssensor 4 sämtliche alltäglichen Bewegungsmuster erfaßt werden, welche zu einem Energieverbrauch im Lebewesen führen. Beispielsweise kann der Bewegungssensor 4 Mittel zur Erfassung einer Beschleunigung, einer Geschwindigkeit oder einer Positionsänderung aufweisen. Bei der Messung der Positionsänderung bzw. Beschleunigung erfolgt die Berechnung in eine Bewegung vorzugsweise über eine zeitliche Integration, so daß beispielsweise die zurückgelegte Wegstrecke ermittelt werden kann. Der Bewegungssensor 4 kann vorzugsweise eine Speichereinrichtung sowie eine Zeiterfassungseinrichtung aufweisen. Infolgedessen kann das erfindungsgemäße Monitoring-System so ausgebildet sein, daß der Bewegungssensor 4 lediglich zu vorgegebenen Zeitpunkten mit der Datenverarbeitungseinrichtung 2 in Signalverbindung tritt, und dieser die erfaßten bzw. ermittelten Daten übermittelt (d.h. zeit-versetzt). Alternativ kann jedoch der Bewegungssensor 4 kontinuierlich mit der Datenverarbeitungseinrichtung 2 in Signalverbindung stehen, so daß die Übermittlung der mit den erfaßten Bewegungsmustern in Zusammenhang stehenden Daten (Bewegungsmusterdaten) kontinuierlich erfolgt. Die Verbindung zwischen Bewegungssensor 4 und Datenverarbeitungseinrichtung 2 bzw. zwischen Datenverarbeitungseinrichtung 2 und Feedback-Einrichtung 6 kann kabelgebunden sein, jedoch ebenfalls über Funkübertragungsmittel erfolgen, wie später mit Fig. 2 erläutert wird. Bei der kabelgebundenen Signalverbindung kann die Verbindung über eine serielle Schnittstelle, eine USB-Schnittstelle oder ähnlichem erfolgen. Da der Bewegungssensor 4 am Lebewesen anordenbar ist, ist dieser vorteilhafterweise in seinen geometrischen Abmessungen so gestaltet, daß er ein Volumen kleiner als 0,5 m³, vorzugsweise kleiner als 0,05 m³ und besonders bevorzugterweise kleiner als 0,001 m³, umfaßt. Ebenfalls ist der Bewegungssensor 4 leichter als 0,5 kg, vorzugsweise leichter 0,1 kg und besonders bevorzugterweise leichter als 0,05 kg.

In Fig. 2 ist eine alternative Ausführungsform des erfindungsgemäßen Monitoring-Systems dargestellt, wobei die zur ersten Ausführungsform identischen Elemente mit den gleichen Bezugsziffern versehen wurden. Die Bewegungssensoren 4 können zur Erfassung des Bewegungsmusters ein Sensorelement 16 aufweisen. Wie bereits dargelegt, kann das Sensorelement 16 vorteilhafterweise die Beschleunigung, Geschwindigkeit oder Position messen. Ebenfalls ist es möglich, ein Sensorelement 16 vorzusehen, welches die Drehrate erfassen kann. Das Sensorelement 16 des Bewegungssensors 4 ist vorteilhafterweise mit Funkübertragungsmitteln 18 versehen, welche eine Signalverbindung über entsprechende Funkübertragungsmittel 20 mit dem Komparator 10 bereitstellen. Entsprechend kann auch die Steuereinheit 14 über Funkübertragungsmittel 22 und 24 mit der Feedback-Einrichtung 6 in Signalverbindung stehen. Die Datenverarbeitungseinrichtung 2 umfaßt weiterhin eine Zeiterfassungseinrichtung 26. Mittels der Zeiterfassungseinrichtung 26 ist es vorteilhafterweise möglich, zu ermitteln, zu welchem Zeitpunkt ein Bewegüngsmuster durch den Bewegungssensor 4 erfaßt wurde. Infolgedessen kann nicht nur in Abhängigkeit des Vergleichsergebnisses des Komparators 10 sondern auch in Abhängigkeit der Zeiterfassungseinrichtung 26 durch die Prozessoreinheit 8 bestimmt werden, ob ein positives Feedback über die Feedback-Einrichtung 6 zu geben ist. Beispielsweise kann die Prozessoreinrichtung 8 derart ausgebildet sein, daß zur Ausgabe eines positiven Feedbacks über die Feedback-Einrichtung 6 ein qualitativ und/oder quantitativ positives Vergleichsergebnis zu vorbestimmten Tageszeiten zu erfolgen hat, beispielsweise morgens, mittags und abends. Somit kann gewährleistet werden, daß sich das Lebewesen kontinuierlich über einen Tageszeitraum bewegt. Darüber hinaus kann das erfindungsgemäße Monitoring-System bevorzugterweise eine Eingabeeinrichtung 28 aufweisen. Über die Eingabeeinrichtung 28 können weitere Eingabeparameter eingegeben werden. Derartige Eingabeparameter können beispielsweise die Größe, das Gewicht, das Alter oder der Gesundheitszustand des Lebewesens sein. Darüber hinaus könnte über die Eingabeeinrichtung 28 das Ernährungsverhalten bzw. die Energiebilanz eingegeben werden, insbesondere-die Auswahl und Zusammenstellung der Nahrungszufuhr. Ferner kann die Eingabeeinrichtung ausgelegt sein, ebenfalls Eingabemöglichkeiten für sportliche Betätigungen vorzusehen, welche in der Eingabeeinrichtung 28 hinterlegt sind. So wäre es z.B. denkbar, in der Eingabeeinrichtung 28 eine Eingabemöglichkeit für z.B. Schwimmen einzugeben, sofern de Bewegungssensor 4 lediglich in der Lage ist, zu erkennen, ob das Lebewesen sitzt, steht oder liegt, da konsequenterweise der Bewegungssensor somit erkennen würde, daß die Person läge, und sich nicht bewegt, so daß kein Energieverbrauch stattfände. Infolgedessen dient die Eingabeeinrichtung 28 vorteilhafterweise zur Eingabe von Bewegungsmustern, welche nicht durch den oder die Bewegungssensoren 4 erfaßt werden können.

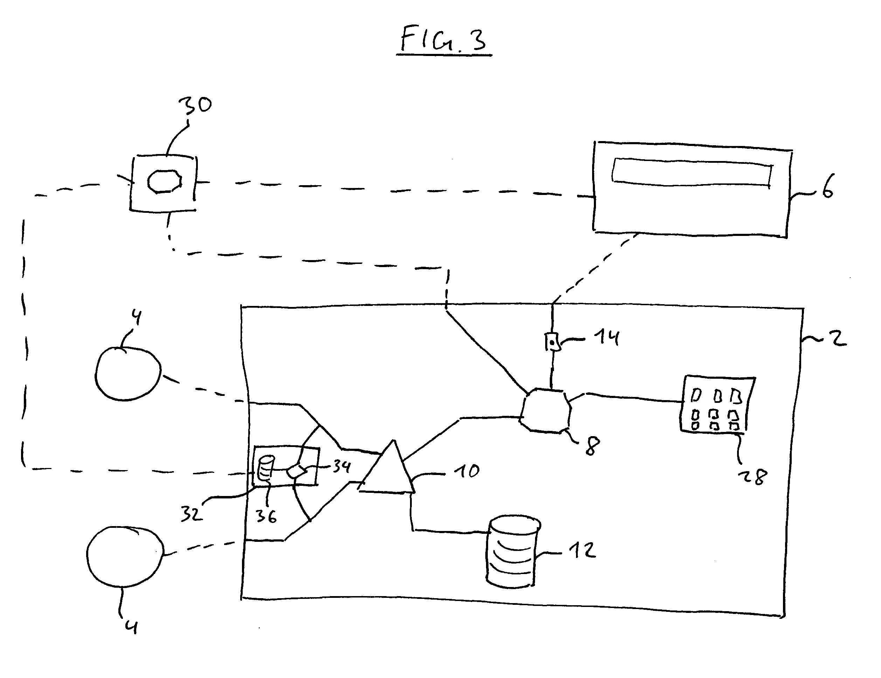

In Fig. 3 ist eine alternative Ausführungsform des erfindungsgemäßen Monitoring-Systems dargestellt, wobei die mit dem ersten und zweiten Ausführungsbeispiel identischen Elemente mit identischen Bezugszeichen versehen sind. Zusätzlich weist das Monitoring-System eine Auswerteeinrichtung 30 auf. Die vorzugsweise externe Auswerteeinrichtung 30 ist mit einer vorzugsweise internen, d.h. in der Datenverarbeitungseinrichtung 2 vorgesehenen, Gesundheitsdatenbank 32, verbunden. Die Gesundheitsdatenbank 32 umfaßt Klassifizierungsmittel 34 und eine Speichereinrichtung 36. Die Klassifizierungsmittel 34 der Gesundheitsdatenbank 32 sind vorteilhafterweise ausgelegt, die mit den von den Bewegungssensoren 4 erfaßten Bewegungsmusterdaten zu erfassen und entsprechend medizinisch zu klassifizieren. In anderen Worten sind die Klassifizierungsmittel 34 ausgelegt, die Bewegungsmuster des Lebewesens zu erkennen, zeitlich zuzuordnen und, entsprechend einer medizinischen Klassifizierungsvorgabe zu ordnen. Diese klassifizierten Daten können dann in der Speichereinrichtung 36 abgelegt werden und/oder über eine Signalverbindung der Auswerteeinrichtung 30 zugeführt werden. Über die Auswerteeinrichtung 30 ist es vorteilhafterweise für Dritte (beispielsweise einen Arzt) möglich, die Bewegung eines Lebewesens zu überwachen. Über die Signalverbindung der Auswerteeinrichtung 30 mit der Prozessoreinrichtung 8 ist es somit möglich, die Datenverarbeitungseinrichtung 2 extern zu steuern, beispielsweise einem geänderten Gesundheitszustand anzupassen. Darüber hinaus ist es ebenfalls möglich, daß über eine Signalverbindung zwischen der Auswerteeinrichtung 30 und der Feedback-Einrichtung 6 ein gesteuertes Feedback dem Lebewesen übermittelt werden kann. Somit stellt die Auswerteeinrichtung 30 eine zweite Instanz für die Ausgabe eines Feedbacks über die Feedback-Einrichtung 6 dar. Es versteht sich, daß die Signalverbindung zwischen den Elementen des erfindungsgemäßen Monitor-Systems, insbesondere zwischen der Auswerteeinrichtung 30 und der Datenverarbeitungseinrichtung 2 bzw. der Feedback-Einrichtung 6, auch über das Internet erfolgen kann.

### Bezugszeichenliste

- 2: Datenverarbeitungseinrichtung
- 4: Bewegungssensor
- 6: Feedback-Einrichtung
- 8: Prozessoreinheit
- 10: Komparator
- 12: Datenbank
- 14: Steuereinheit
- 16: Sensorelement
- 18, 20, 22, 24: Funkübertragungsmittel
- 26: Zeiterfassungseinrichtung
- 28: Eingabeeinrichtung
- 30: Auswerteeinrichtung
- 32: Gesundheitsdatenbank
- 34: Klassifizierungsmittel
- 36: Speichereinrichtung

## Patentansprüche

1. Monitoring System, insbesondere Adipositas-Monitoring System, zur Überwachung der Aktivität eines Lebewesens, umfassend
zumindest einen Bewegungssensor (4), der am Lebewesen anordenbar und ausgelegt ist, alltägliche Bewegungsmuster zu erfassen,
zumindest eine Feedbackeinrichtung (6), welche am Lebewesen oder diesem benachbart anordenbar ist, und
zumindest eine Datenverarbeitungseinrichtung (2), welche mobil ausgebildet ist und mit dem Bewegungssensor (4) und der Feedbackeinrichtung (6) in Signalverbindung steht,
wobei die Datenverarbeitungseinrichtung (2) aufweist
- eine Datenbank (12), welche Daten in Form von vorgegebenen Bewegungsmustern umfaßt,
- einen Komparator (10), der zum Vergleich der vom Bewegungsensor (4) ermittelten Bewegungsmuster mit den vorgegebenen Bewegungsmustern der Datenbank (12) ausgelegt ist, und
- eine Prozessoreinheit (8), welche mit dem Komparator (10) in Signalverbindung steht und ausgelegt ist, eine Steuereinheit (14) der Datenverarbeitungseinrichtung (2) in Abhängigkeit des Vergleichsergebnisses zu steuern bzw. zu regeln, wobei die Steuereinheit (14) ausgelegt ist, innerhalb einer vorbestimmbaren Zeitdauer in Abhängigkeit der Steuerung bzw. Regelung durch die Prozessoreinheit (8) eine für das Lebewesen wahrnehmbare Reaktion über die Feedbackeinrichtung (6) auszuüben.

2. Monitoring System nach Anspruch 1, wobei der Bewegungssensor (4) Mittel zur Erfassung einer Beschleunigung aufweist, um über eine zeitliche Integration ein Bewegungsmuster zu erfassen.

3. Monitoring System nach einem der vorhergehenden Ansprüche, wobei der Bewegungssensor (4) Mittel zur Erfassung einer Geschwindingkeit aufweist, um ein Bewegungsmuster zu erfassen.

4. Monitoring System nach einem der vorhergehenden Ansprüche, wobei der Bewegungssensor (4) Mittel zur Erfassung der Position aufweist, um über eine zeitliche Integration bei Positionsänderung ein Bewegungsmuster zu erfassen.

5. Monitoring System nach einem der vorhergehenden Ansprüche, wobei die Datenverarbeitungseinrichtung (2) und/oder der Bewegungssensor (4) eine Zeiterfassungseinrichtung aufweisen, um die Bewegungsmuster über einen vorbestimmten oder vorbestimmbaren Zeitraum zu erfassen.

6. Monitoring System nach einem der vorhergehenden Ansprüche, weiterhin aufweisend eine Eingabeeinrichtung (28) zur Eingabe von mit Bewegungsmustern in Zusammenhang stehenden Daten.

7. Monitoring System nach einem der vorhergehenden Ansprüche, weiterhin aufweisend eine Speichereinrichtung, die vorzugsweise in der Datenverarbeitungseinrichtung (2), dem Bewegungssensor (4) und/oder der Feedbackeinrichtung (6) vorgesehen ist.

8. Monitoring System nach einem der vorhergehenden Ansprüche, wobei die Feedbackeinrichtung (6) optische, akustische und/oder kinästhetische Ausgabemittel umfaßt, um eine Reaktion auszuüben.

9. Monitoring System nach einem der vorhergehenden Ansprüche, wobei zumindest die Feedbackeinrichtung (6) und die Datenverarbeitungseinrichtung (2) integral ausgebildet sind.

10. Monitoring System nach einem der vorhergehenden Ansprüche, weiterhin aufweisend eine Auswerteeinrichtung (30), welche mit der Datenverarbeitungseinrichtung (2) in Signalverbindung steht oder biringbar ist, um die erfaßten Bewegungsmuster und/oder die Vergleichsergebnisse weiterzuverarbeiten.

11. Monitoring System nach Anspruch 10, wobei die Datenverarbeitungseinrichtung (2) und/oder die Feedbackeinrichtung (6) ausgelegt sind, von der Auswerteeinrichtung (30) Daten zu empfangen.

12. Monitoring System nach einem der vorhergehenden Ansprüche, wobei die Datenverarbeitungseinrichtung (2) eine weitere Datenbank in Form einer Gesundheitsdatenbank (32) aufweist, welche ausgelegt ist, die durch den Bewegungssensor (4) erfaßten Bewegungsmuster medizinisch zu klassifizieren und/oder zu speichern.

13. Monitoring System nach Anspruch 12, wobei die weitere Datenbank (32) ausgelegt ist, die klassifizierten Daten an die Auswerteeinrichtung (30) zu übermitteln.

14. Monitoring System nach einem der vorhergehenden Ansprüche, wobei die Datenverarbeitungseinrichtung (2), der Bewegungssensor (4), die Feedbackeinrichtung (6) und/oder die Auswerteeinheit (30) Funkübertragungsmittel (18, 20, 22, 24) aufweisen.

15. Monitoring Verfahren, insbesondere Adipositas-Monitoring Verfahren, zur Überwachung der Aktivität eines Lebewesens, umfassend die Schritte:
- Erfassen eines alltäglichen Bewegungsmusters des Lebewesens mittels eines am Lebewesen anordenbaren Bewegungssensor (4),
- Übertragen von dem erfaßten Bewegungsmuster entsprechenden Daten an eine mobile Datenverarbeitungseinrichtung (2)
- Vergleichen der erfaßten Bewegungsmusterdaten mit in einer Datenbank (12) der Datenverarbeitungseinrichtung (2) abgelegten vorgegebenen Bewegungsmusterdaten durch einen Komparator (10)
- Steuern bzw. Regeln einer Steuereinheit (14) der Datenverarbeitungseinrichtung (2) in Abhängikeit des Vergleichsergebnisses
- Ausüben einer für das Lebewesen wahrnehmbaren Reaktion durch die Feedbackeinrichtung (6) innerhalb einer vorbestimmbaren Zeitdauer über eine Steuereinheit (14) in Abhängigkeit der Steuerung bzw. Regelung durch die Prozessoreinheit (8).

16. Monitoring Verfahren nach Anspruch 15, weiterhin aufweisend den Schritt:
Übertragen der erfaßten Bewegungsmusterdaten und/oder des Vergleichsergebnisses an eine mit der Datenverarbeitungseinrichtung (2) in Signalverbindung stehenden Auswerteeinrichtung (30).

17. Monitoring Verfahren nach Anspruch 16, weiterhin aufweisend den Schritt:
Übertragen von Daten von der Auswerteeinrichtung (30) an die Datenverarbeitungseinrichtung (2) und/oder Feedbackeinrichtung (6).

18. Monitoring Verfahren nach einem der Ansprüche 15 - 17, weiterhin aufweisend den Schritt:
Medizinisch Klassifizieren und/oder Speichern der erfaßten Bewegungsmusterdaten in einer Gesundheitsdatenbank (32) der Datenverarbeitungseinrichtung (2).
